# EUROPEAN PATENT APPLICATION

(11) **EP 4 649 894 A1**
(43) Date of publication of application: **19.11.2025**
(21) Application number: 25176229.0
(22) Date of filing: 13.05.2025
(51) Int. Cl.: A61B 8/06, A61B 8/00, A61B 8/08

(54) **ULTRASONIC DIAGNOSIS DEVICE, MEDICAL INFORMATION PROCESSING DEVICE, AND PROGRAM**

(30) Priority: 14.05.2024 JP 2024078994; 12.05.2025 JP 2025080033
(71) Applicant: Canon Medical Systems Corporation, Tochigi 324-0036 (JP)
(72) Inventor: USUMURA, Masashi, Otawara-shi, 324-0036 (JP); IGARASHI, Seito, Otawara-shi, 324-0036 (JP)
(74) Representative: Marks & Clerk LLP

(57) **Abstract**

A medical information processing device according to an embodiment includes an ultrasound probe (5) and processing circuitry(110). The processing circuitry(110) obtains first ultrasound data obtained by execution of an ultrasound scan of a subject via the ultrasound probe (5), the first ultrasound data representing plural frames consecutive in a time direction; inputs data based on the first ultrasound data into a trained model; and obtains second ultrasound data from the trained model, the second ultrasound data representing a single frame and being higher in spatial resolution of fluid in the subject than the first ultrasound data.

## Description

### FIELD

Embodiments disclosed in this specification and the drawings are related to an ultrasonic diagnosis device, a medical information processing device, and a program.

### BACKGROUND

Ultrasonic diagnosis devices have been widely used for observation and diagnosis of blood flows in living bodies. Ultrasonic diagnosis devices generate and display blood flow information from reflected ultrasound waves using the Doppler method based on the Doppler effect. Blood flow information generated and displayed by ultrasonic diagnosis devices includes color Doppler images and Doppler waveforms (Doppler spectra).

Color Doppler images are imaged by the color flow mapping (CFM) method. In the CFM method, transmission and reception of ultrasound are performed on plural scanning lines a plural number of times. By application of a moving target indicator (MTI) filter to data sequences for the same position, signals (clutter signals) originating from stationary tissue or slow-moving tissue are reduced and signals originating from a blood flow are extracted. In the CFM method, blood flow information, such as values of velocity, variance, and power of the blood flow, is estimated from the blood flow signals and distributions of results of this estimation are displayed as Doppler images.

B-mode data and Doppler data are known to be reduced in image quality according to a point spread function (PSF) determined by, for example, the wavelength of transmitted ultrasound and the transmission and reception aperture width. One solution is to increase the frequency of the transmitted ultrasound, but there are limitations to the frequency band of the probe.

Generating blood flow data improved in resolution by respectively extracting parts of plural sets of blood flow data that are consecutive in the time direction is described in Non-Patent Literature 1, the parts being high in signal value (amplitude value). More specifically, utilizing the fact that amplitude distribution characteristics of a speckle pattern of blood flow data is able to be roughly approximated by a probability distribution called a Rayleigh distribution is described in Non-Patent Literature 1. Spatially sparse signals large in signal value (amplitude value) and high in luminance (high in resolution), but small in probability of occurrence are thereby extracted from the respective sets of blood flow data and are added up.

However, in this method described in Non-Patent Literature 1, when a large number of frames are used, the computational processing load is increased, and the responsiveness may thus be decreased and the real-time performance of the ultrasonic diagnosis device may thus be degraded.

### SUMMARY OF INVENTION

An ultrasonic diagnosis device according to an aspect of the present invention comprises an ultrasound probe and processing circuitry. The processing circuitry obtains first ultrasound data obtained by execution of an ultrasound scan of a subject via the ultrasound probe, the first ultrasound data representing plural frames consecutive in a time direction. The processing circuitry inputs data based on the first ultrasound data into a trained model to obtain second ultrasound data from the trained model, the second ultrasound data representing a single frame and being higher in spatial resolution of fluid in the subject than the first ultrasound data.

The processing circuitry may input, as the first ultrasound data, at least one of: (1) IQ data corresponding to the plural frames and before application of a wall filter; (2) Doppler data that correspond to the plural frames and are the IQ data after the application of the wall filter; (3) power Doppler data corresponding to the plural frames and representing absolute values of the Doppler data; and (4) data corresponding to the plural frames and resulting from local peak extraction processing of the power Doppler data, into the trained model, and obtain, as the second ultrasound data, ultrasound data corresponding to data resulting from combination processing of data corresponding to the plural frames and resulting from local peak extraction processing, from the trained model.

The processing circuitry may input, as the first ultrasound data, data that are a combination of two or more of: (1) the IQ data; (2) the Doppler data; (3) the power Doppler data; and (4) the data resulting from the local peak extraction processing, into the trained model, the data corresponding to the plural frames.

The processing circuitry may input, as the first ultrasound data, data that are a combination of (1) the IQ data and (2) the Doppler data, into the trained model, the data corresponding to the plural frames.

The processing circuitry may input, as the first ultrasound data, data that are a combination of (1) the IQ data and (3) the power Doppler data, into the trained model, the data corresponding to the plural frames.

The processing circuitry may input, as the first ultrasound data, data that are a combination of (2) the Doppler data and (3) the power Doppler data, into the trained model, the data corresponding to the plural frames.

The processing circuitry may input, as the first ultrasound data, data that are a combination of (1) the IQ data, (2) the Doppler data, and (3) the power Doppler data, into the trained model, the data corresponding to the plural frames.

A medical information processing device according to the present invention comprises processing circuitry. The processing circuitry obtains first ultrasound data obtained by execution of an ultrasound scan of a subject via an ultrasound probe, the first ultrasound data representing plural frames consecutive in a time direction, and inputs data based on the first ultrasound data into a trained model to obtain second ultrasound data from the trained model, the second ultrasound data representing a single frame and being higher in spatial resolution of fluid in the subject than the first ultrasound data.

A program according to an aspect of the present invention causing a computer to execute a process including:
obtaining first ultrasound data obtained by execution of an ultrasound scan of a subject via an ultrasound probe, the first ultrasound data representing plural frames consecutive in a time direction; and inputting data based on the first ultrasound data into a trained model and obtaining second ultrasound data from the trained model, the second ultrasound data representing a single frame and being higher in spatial resolution of fluid in the subject than the first ultrasound data.

### BRIEF DESCRIPTION OF THE DRAWINGS

FIG. 1 is a block diagram illustrating a configuration of an ultrasonic diagnosis device according to an embodiment;
FIG. 2 is a diagram for description of a Rayleigh distribution;
FIG. 3 is a diagram illustrating a processing procedure to obtain a high-resolution signal;
FIG. 4 is a diagram for description of processing performed in a training phase by a medical information processing device according to a first embodiment;
FIG. 5 is a diagram for description of processing performed by the medical information processing device according to the first embodiment;
FIG. 6A is a diagram illustrating a conventional ultrasound image;
FIG. 6B is a diagram illustrating an ultrasound image represented by second ultrasound data;
FIG. 7 is a diagram for description of processing performed by a medical information processing device according to a second embodiment;
FIG. 8 is a diagram for description of processing performed by a medical information processing device according to a third embodiment;
FIG. 9 is a diagram for description of processing performed by a medical information processing device according to a fourth embodiment;
FIG. 10 is a diagram for description of processing performed by a medical information processing device according to a fifth embodiment;
FIG. 11 is a diagram for description of processing performed by a medical information processing device according to a sixth embodiment;
FIG. 12 is a diagram for description of processing performed by a medical information processing device according to a seventh embodiment;
FIG. 13 is a diagram for description of processing performed by a medical information processing device according to an eighth embodiment;
FIG. 14 is a diagram for description of the processing performed by the medical information processing device according to the eighth embodiment;
FIG. 15 is a diagram for description of the processing performed by the medical information processing device according to the eighth embodiment;
FIG. 16 is a diagram for description of the processing performed by the medical information processing device according to the eighth embodiment; and
FIG. 17 is a diagram for description of the processing performed by the medical information processing device according to the eighth embodiment.

### DETAILED DESCRIPTION

A medical information processing device provided in one aspect of the present invention includes an ultrasound probe and processing circuitry. The processing circuitry obtains first ultrasound data obtained by execution of an ultrasound scan of a subject via the ultrasound probe, the first ultrasound data representing plural frames consecutive in a time direction, inputs data based on the first ultrasound data into a trained model, and obtains second ultrasound data from the trained model, the second ultrasound data representing a single frame and being higher in spatial resolution of fluid in the subject than the first ultrasound data.

### First Embodiment

Embodiments of a medical information processing device, an ultrasonic diagnosis device, and a program will be described in detail hereinafter while reference is made to the drawings.

An ultrasonic diagnosis device according to an embodiment causes an ultrasound probe to execute an ultrasound scan and collects plural sets of frame data obtained by execution of the ultrasound scan, the plural sets of frame data being consecutive in a time direction (plural sets of frame data in a predetermined time period). The frame data are collected at a predetermined frame rate by the execution of the ultrasound scan. The frame data refer to any of received data, measured data, blood flow data, and tissue data. The received data are, for example, received ultrasound signals (for example, channel data) received by the ultrasound probe. The measured data are data (for example, IQ data) obtained by phasing addition and quadrature detection processing of the received ultrasound signals. The blood flow data are data (for example, power Doppler signal data) resulting from extraction or enhancement of information in the measured data, the information originating from a blood flow. The tissue data are data (for example, B-mode data) resulting from extraction or enhancement of information originating from tissue.

The measured data include, for example, the information originating from the tissue (tissue signal component (clutter)) and the information originating from the blood flow (blood flow signal component). The information originating from the blood flow may include, not only information originating from blood, but also information originating from a contrast agent in the blood. Furthermore, the blood flow data are data resulting from extraction or enhancement of the information originating from the blood flow and include values of velocity, variance, and power of the blood flow.

The extraction of the information originating from the blood flow is, for example, operation to take out the blood flow signal component from the measured data. The enhancement of the information originating from the blood flow is, for example, processing to accentuate the blood flow signal component more in relation to the tissue signal component. The blood flow data may be obtained by processing to extract or enhance the information originating from the blood flow or may be obtained by processing to remove or reduce the information originating from the tissue.

An ultrasonic diagnosis device will be described as an example with respect to this embodiment, but the embodiment may be applied to a modality (medical information processing device) other than the ultrasonic diagnosis device. For example, the embodiment may be applied to a medical information processing device, such as a workstation or a server, which obtains ultrasound data obtained on the basis of a result of an ultrasound scan of a subject.

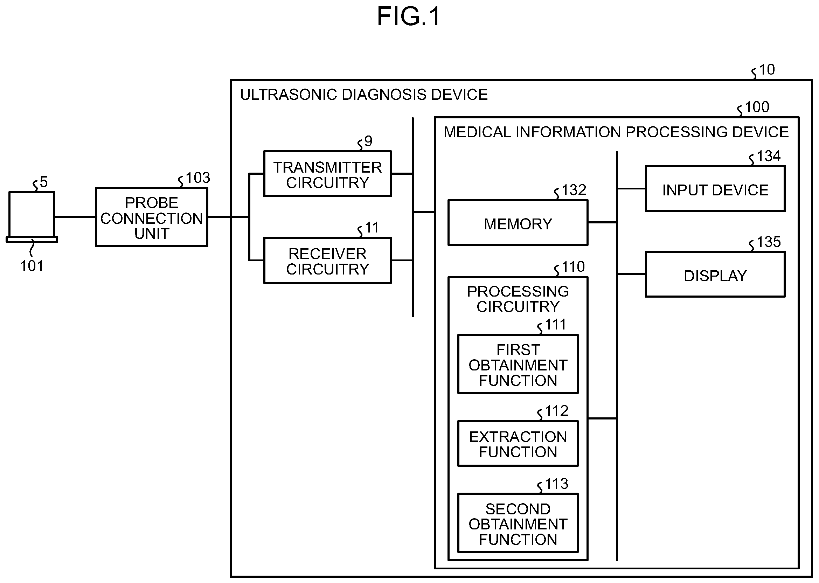

FIG. 1 is a block diagram illustrating a configuration of an ultrasonic diagnosis device according to an embodiment. An ultrasonic diagnosis device 10 is a device that generates ultrasound data on the basis of received signals (reflected wave signals) received from an ultrasound probe 5. The ultrasonic diagnosis device 10 illustrated in FIG. 1 is a device capable of generating two-dimensional ultrasound data on the basis of two-dimensional received signals and capable of generating three-dimensional ultrasound data on the basis of three-dimensional received signals. However, the embodiment is also applicable to a case where the ultrasonic diagnosis device 10 is a device dedicated to two-dimensional data. The ultrasonic diagnosis device 10 includes transmitter circuitry 9, receiver circuitry 11, and a medical information processing device 100.

The ultrasound probe 5 is, for example, an electronic scanning probe, and has plural transducers 101 arranged one-dimensionally or two-dimensionally at a distal end thereof. The transducers 101 are piezoelectric elements (electromechanical transducers) that perform mutual conversion between electric signals (voltage pulse signals) and ultrasound (acoustic waves). The ultrasound probe 5 transmits ultrasound from the plural transducers 101 to a subject and receives reflected ultrasound from the subject by means of the plural transducers 101. The reflected acoustic waves reflect differences between acoustic impedances within the subject. In a case where ultrasound pulses transmitted are reflected by a moving blood flow or a surface of a heart wall, for example, reflected ultrasound experiences a frequency shift due to the Doppler effect, the frequency shift being dependent on a velocity signal component of the moving object in relation to the ultrasound transmission direction.

A probe connection unit 103 connects the ultrasound probe 5 and transmits and receives ultrasound to and from the ultrasound probe 5. The probe connection unit 103 may connect the ultrasound probe 5 by a wired or wireless means. In the wired case, the probe connection unit 5 has a connector unit (receptacle) to connect a connector (plug) of the ultrasound probe 5. In the wireless case, a communication unit to perform wireless communication with the ultrasound probe 5 is included.

The transmitter circuitry 9 is a transmitter unit that outputs pulse signals (drive signals) to the plural transducers 101. Applying pulse signals to the plural transducers 101 with time differences causes the plural transducers 101 to transmit ultrasound with different time delays and a transmitted ultrasound beam is thereby formed. Selectively changing the transducers 101 (that is, the transducers 101 to be driven) that the pulse signals are to be applied to and changing the time delays (application times) of the pulse signals enable control of the direction and focus of the transmitted ultrasound beam. Successively changing the direction and focus of the transmitted ultrasound beam allows an observed region inside the subject to be scanned. Furthermore, a transmitted ultrasound beam having plane waves (with a far focus) or diffuse waves (with a focus point in a direction opposite to the ultrasound transmission direction in relation to the plural transducers 101) may be formed by changing the time delays of the pulse signals. Alternatively, one transducer or some of the plural transducers 101 may be used to form a transmitted ultrasound beam. By transmitting pulse signals having a predetermined drive waveform to the transducers 101, the transmitter circuitry 9 causes transmitted ultrasound to be generated in the transducers 101, the transmitted ultrasound having a predetermined transmission waveform.

The receiver circuitry 11 is a receiver unit that inputs, as received signals, electric signals output from the transducers 101 that have received reflected ultrasound. The received signals are input to processing circuitry 110. With respect to this embodiment, analog signals output from the transducers 101 and digital data sampled (digitally converted) from the analog signals will be referred to as received signals with no particular distinction. However, for the purpose of clarifying that received signals are digital data according to context, received signals may be referred to as received data or measured data.

The medical information processing device 100 is connected to the transmitter circuitry 9 and the receiver circuitry 11 and executes processing of signals received from the receiver circuitry 11 and control of the transmitter circuitry 9. The medical information processing device 100 includes the processing circuitry 110, a memory 132, an input device 134, and a display 135.

The memory 132 includes, for example, a semiconductor memory element, such as a random access memory (RAM) or a flash memory, a hard disk, and an optical disk. The memory 132 is a memory to store data, such as image data for display, the image data having been generated by the processing circuitry 110. Furthermore, the memory 132 is also capable of storing received signals (reflected wave signals) output by the receiver circuitry 11. In addition, as required, the memory 132 stores: control programs for performing ultrasound transmission and reception, image processing, and display processing; and various data, such as diagnostic information (for example, patient IDs and medical doctors' findings), diagnostic protocols, and various body marks.

The input device 134 receives various instructions and input of information from an operator. The input device 134 includes, for example, input interface devices, such as a mouse, a keyboard, a button, and a trackball.

Under control by the processing circuitry 110, the display 135 displays: a graphical user interface (GUI) for receiving input of imaging conditions; and various images. The display 135 includes, for example, a display interface device, such as a liquid crystal display.

By controlling each unit of the ultrasonic diagnosis device 10, the processing circuitry 110 controls the whole ultrasonic diagnosis device 10. The processing circuitry 110 is described as being implemented by a single processor in FIG. 1, but plural independent processors may be combined for implementation by the multiple processors. Furthermore, the processing circuitry 110 may include an independent circuit dedicated to a specific function, such as an application specific integrated circuit (ASIC).

Furthermore, the term, "processor", used in the above description means, for example: a central processing unit (CPU); a graphical processing unit (GPU); or a circuit, such as an application specific integrated circuit (ASIC) or a programmable logic device (for example, a simple programmable logic device (SPLD), a complex programmable logic device (CPLD), and a field programmable gate array (FPGA)). The processor implements functions by reading and executing the programs stored in the memory 132.

The processing circuitry 110 causes the ultrasound probe 5 to execute an ultrasound scan and collects plural sets of frame data obtained by execution of the ultrasound scan, the plural sets of frame data being consecutive in a time direction (plural sets of frame data in a predetermined time period). The processing circuitry 110 performs phasing addition and quadrature detection processing of received signals (channel data) collected via the receiver circuitry 11. The phasing addition processing is processing to add up the received signals from the plural transducers 101 by varying time delays and weights for the respective transducers 101 and is also called delay and sum (DAS) beam forming. The quadrature detection processing is processing to convert the received signals to in-phase signals and quadrature signals (IQ data (measured data)) in a baseband band. In addition, the received signals may be subjected to, for example, adaptive beam forming, model-based processing, or processing using machine learning.

Furthermore, the processing circuitry 110 may estimate an amount of displacement of tissue resulting from, for example, body movement of the subject between the plural sets of frame data and correct each set of frame data on the basis of a result of this estimation. Specifically, the processing circuitry 110 calculates an amount of displacement of tissue resulting from, for example, body movement between frames, from the plural sets of frame data. The processing circuitry 110 corrects the frame data on the basis of the amount of displacement calculated.

Furthermore, the processing circuitry 110 performs, for example, envelope detection processing and logarithmic compression processing to generate B-mode data (data resulting from extraction or enhancement of information originating from tissue) representing, as luminance, signal intensities at respective points in an observed region. Furthermore, the processing circuitry 110 generates blood flow data (power Doppler signal data) resulting from extraction or enhancement of information originating from a blood flow in the measured data.

For example, the processing circuitry 110 applies a moving target indicator (MTI) filter to the plural sets of frame data. Information (tissue signal component (clutter)) originating from tissue remaining stationary or tissue small in movement between frames is thereby reduced and the information originating from the blood flow (blood flow signal component) is thus extracted. A filter having a fixed filter coefficient, such as a Butterworth infinite impulse response (IIR) filter or a polynomial regression filter, may be used as the MTI filter. The MTI filter may be an adaptive filter that changes the coefficient according to input signals by using eigenvalue decomposition or singular value decomposition.

Furthermore, the processing circuitry 110 may decompose frame data into plural bases using eigenvalue decomposition or singular value decomposition, remove the information originating from the tissue by taking out specific bases, and extract the information originating from the blood flow. Furthermore, the processing circuitry 110 may find the velocity vector for each coordinate in received signal data by using a method, such as the vector Doppler method, the speckle tracking method, or the vector flow mapping method, to find a blood flow vector representing the magnitude and direction of the blood flow. Any method other than the methods described herein as examples may be adopted, as long as that method enables extraction or enhancement of the information originating from the blood flow included in the frame data (received data or measured data) or removal or reduction of the information originating from the tissue.

Furthermore, by reading and executing the programs stored in the memory 132, the processing circuitry 110 cause a first obtainment function 111, an extraction function 112, and a second obtainment function 113 to function, to perform processing to output high-definition ultrasound data.

A method of generating high-definition ultrasound data according to a conventional technique (for example, Non-Patent Literature 1) will be described before description of processing using the ultrasonic diagnosis device 10 configured as described above.

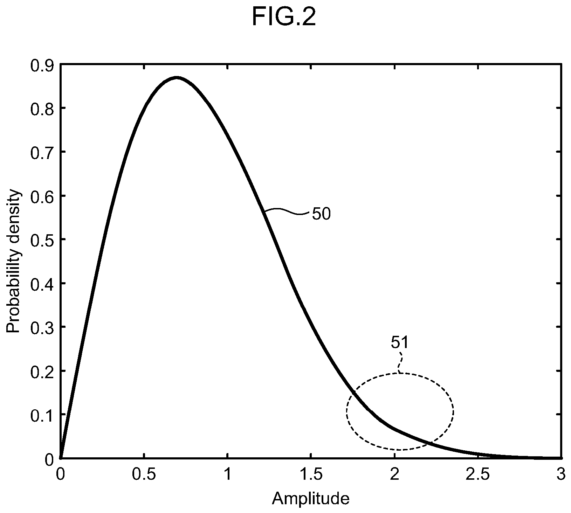

As illustrated in FIG. 2, amplitude distribution characteristics 50 of a speckle pattern of blood flow data (for example, IQ signals after removal of clutter) are able to be roughly approximated by a probability distribution called a Rayleigh distribution. In a method of generating high-resolution data (a high-resolution signal) according to the conventional technique, spatially sparse signals high in signal value and high in luminance (high in resolution) (a portion 51 illustrated in FIG. 2) but small in probability of occurrence are extracted from plural sets of blood flow data respectively corresponding to plural sets of frame data and a high-resolution signal is generated by combining (for example, totaling up or adding up) these spatially sparse signals.

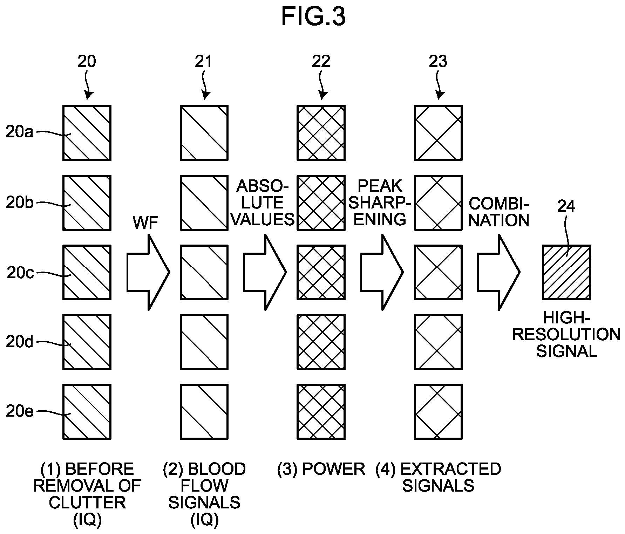

Specifically, as illustrated in FIG. 3, from execution of an ultrasound scan of a subject via an ultrasound probe to obtainment of a high-resolution signal 24, processing to obtain (1) IQ signals 20 before removal of clutter, (2) blood flow signals 21 (IQ signals), (3) power Doppler signals 22, and (4) extracted signals 23 is performed.

The IQ signals 20 are obtained by phasing addition and quadrature detection processing of received signals (reflected ultrasound signals) representing plural frames 20a, 20b, 20c, 20d, and 20e obtained through the ultrasound scan of the subject.

The blood flow signals 21 are signals resulting from the removal of the clutter from the IQ signals 20, and are, for example, blood flow data obtained by application of a wall filter (WF) to the IQ signals 20. The blood flow data are Doppler signals representing the velocity, variance, and power of fluid, such as a blood flow. Furthermore, the blood flow signals 21 correspond respectively to the plural frames 20a, 20b, 20c, 20d, and 20e. The blood flow signals 21 may be obtained by application of an MTI filter to the IQ signals 20.

The power Doppler signals 22 are, for example, power Doppler signals obtained by finding absolute values of the IQ signals (Doppler data) serving as the blood flow signals 21. Furthermore, the power Doppler signals 22 correspond respectively to the plural frames 20a, 20b, 20c, 20d, and 20e.

The extracted signals 23 are signals representing a target object and are obtained from the power Doppler signals 22. The target object is, for example, a blood flow large in signal value (amplitude value) but small in probability of occurrence. Furthermore, the extracted signals 23 correspond respectively to the plural frames 20a, 20b, 20c, 20d, and 20e. The extracted signals 23 result from extraction of high-luminance (high-resolution) and spatially sparse signals. Specifically, the extracted signals 23 are each obtained by peak sharpening, in which a nonlinear function is applied to each position (pixel value) in the frame represented by the power Doppler signal 22. In this case, for example, processing to increase the pixel density and increase the effective resolution by subjecting the power Doppler signals 22 to resampling may be performed. The resampling is, for example, processing of replacing each pixel with plural pixels smaller in size, and signal values corresponding to each pixel after this replacement may be interpolated from the original signal value (the signal value before the replacement) by, for example, bicubic interpolation. Furthermore, the signal values corresponding to each pixel representing the target object may be sharpened by calculating the power (for example, the eighth power or twelfth power) of each position (pixel value) in the frame represented by the power Doppler signal 22 after the resampling. The signal values corresponding to the pixels representing the target object are thereby able to be emphasized more than signal values corresponding to pixels around those pixels and the extracted signals 23 that are high in luminance, are spatially sparse, and represent the target object are thereby obtained from the power Doppler signals 22. The extracted signals 23 may be referred to as local peak signals. Furthermore, a blood flow is described as an example of the target object with respect to this embodiment, but tissue or a contrast agent in the body may be the target object.

The high-resolution signal 24 is obtained by combining (totaling up or adding up, for example) the extracted signals 23 (local peak signals) respectively corresponding to the plural frames 20a, 20b, 20c, 20d, and 20e. The high-resolution signal 24 represents a single frame. Furthermore, the high-resolution signal 24 is a signal that is higher in spatial resolution of the fluid in the subject than the IQ signals 20, blood flow signals 21, power Doppler signals 22, and extracted signals 23 and that is higher in definition of (that images with high definition) the fluid flowing in the subject. The load of the signal processing described above is large and the signal processing also takes time, in particular, for the extracted signals 23 to be obtained.

The method according to the conventional technique thus requires plural sets of processing for the high-resolution signal 24 to be obtained. Therefore, when a large number of frames are used, the real-time characteristic of high-definition ultrasound data may be degraded. For example, the number of frames required for the high-resolution signal 24 to be obtained is about several thousands to about several tens of thousands and the real-time performance is thus low.

The ultrasonic diagnosis device 10 according to this embodiment outputs high-definition ultrasound data using a trained model based on artificial intelligence (AI) and thereby minimizes the amount of computation and maintains the real-time performance required for ultrasound diagnosis.

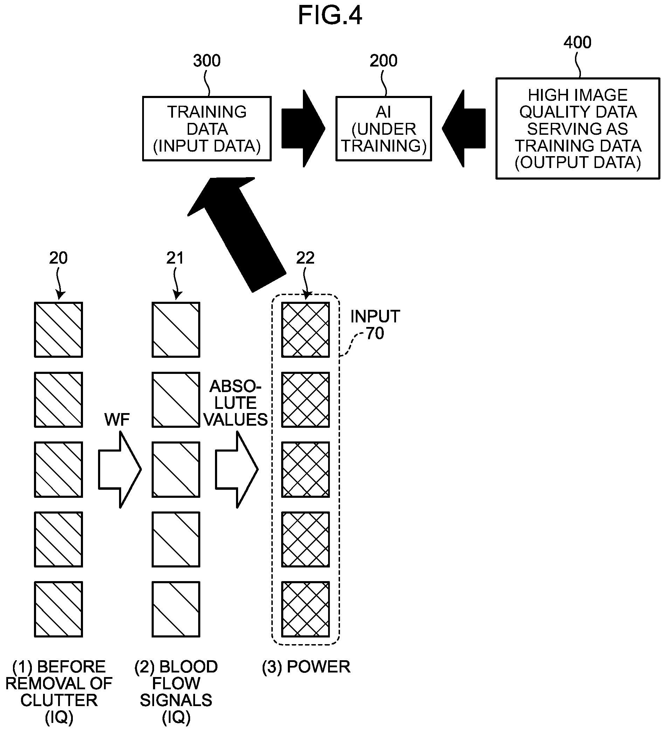

Processing in a training phase will be described hereinafter. In the training phase, training of a neural network is performed with input data 300 and high image quality data 400 serving as output data, the input data 300 and the high image quality data 400 serving as a set of training data. The training of the neural network is performed with: signals (corresponding to data based on first ultrasound data) corresponding to at least one of the above described (1) IQ signals before the removal of the clutter, (2) blood stream signals 21 (IQ signals after the removal of the clutter), (3) power Doppler signals 22, and (4) extracted signals 23; and a signal (corresponding to second ultrasound data) corresponding to the high-resolution signal 24, the signals and the signal serving as one data set. For example, as illustrated in FIG. 4, the training of the neural network is performed with data sets each having power Doppler signals 22 representing plural frames and serving as input data, and high-definition data (a high-resolution signal 24) representing a single frame and serving as output data. In the training of the neural network, model weight coefficients of a trained model 200 are determined. The weight coefficients are determined, by means of a training function not illustrated in the drawings, by calculation of gradients of weights using backpropagation to minimize a given objective function, for example.

Examples of the neural network for the trained model 200 include a perceptron, a single-layer neural network, a CNN, or a deep neural network using a U-Net or a transformer. Furthermore, because the power Doppler signals 22 are sets of data respectively formed of plural frames, the trained model 200 may be a time-series neural network, such as an RNN.

In response to the above described training of the trained model 200, the processing circuitry 110 stores the determined weight coefficients of the neural network into the memory 132. In embodiments other than the first embodiment, processing in their training phases is similar and in each of these embodiments, high image quality data 400 serving as output data are similarly used as training data, but training data used as input data 300 are different.

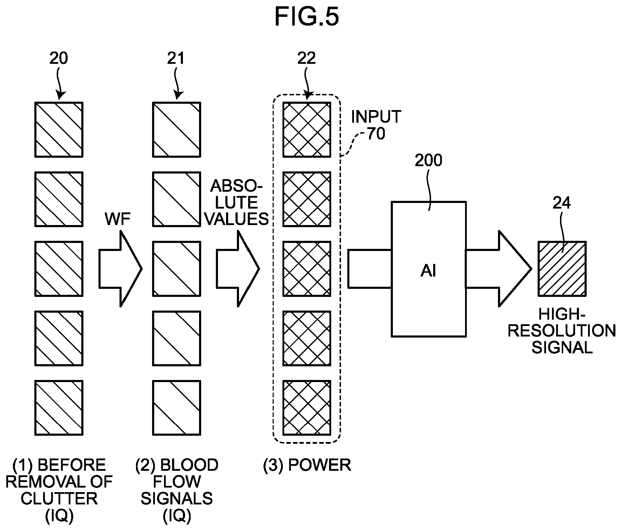

Processing in an operational phase (inference phase) will be described next by reference to FIG. 5. The first obtainment function 111 (first obtainment unit) obtains first ultrasound data obtained on the basis of a result of an ultrasound scan of a subject. That is, the first obtainment function 111 obtains first ultrasound data obtained by execution of an ultrasound scan of a subject via the ultrasound probe 5, the first ultrasound data representing plural frames consecutive in a time direction. The first ultrasound data correspond to received signals (reflected ultrasound signals) representing the plural frames.

The extraction function 112 obtains IQ signals 20 representing the plural frames by performing phasing addition and quadrature detection processing of the received signals (the reflected ultrasound signals) representing the plural frames. Furthermore, the extraction function 112 obtains blood flow signals 21 representing the plural frames by applying a wall filter (WF) to the IQ signals 20. Furthermore, the extraction function 112 obtains power Doppler signals 22 representing the plural frames by finding absolute values of IQ signals (Doppler Data) serving as the blood flow signals 21.

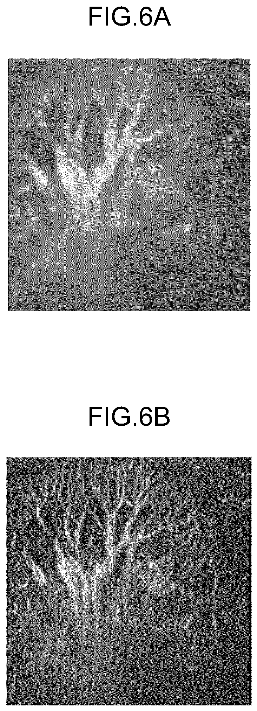

The second obtainment function 113 then inputs data based on the first ultrasound data into the trained model 200. The second obtainment function 113 inputs, as the data based on the first ultrasound data, the power Doppler signals 22 (power Doppler data) obtained by the extraction function 112 and corresponding to the plural frames, into the trained model 200. That is, the power Doppler signals 22 representing the plural frames serve as input 70 to the trained model 200. Furthermore, the second obtainment function 113 obtains second ultrasound data representing a single frame from the trained model 200, the second ultrasound data being higher in spatial resolution of fluid in the subject than the first ultrasound data. The second obtainment function 113 obtains a high-resolution signal 24 (second ultrasound data) as output from the trained model 200. The high-resolution signal 24 corresponds to a signal obtained by combining (totaling up or adding up, for example) local peak signals of the power Doppler signals 22 input to the trained model 200, the power Doppler signals 22 representing the plural frames, and the high-resolution signal 24 represents a single frame. The high-resolution signal 24 is higher in spatial resolution of the fluid in the subject than the received signals, IQ signals 20, blood flow signals 21, and power Doppler signals 22. For example, FIG. 6A illustrates an ultrasound image based on the power Doppler signals 22 and FIG. 6B illustrates an ultrasound image based on the high-resolution signal 24. As illustrated in FIG. 6A and FIG. 6B, the spatial resolution of the fluid (blood flow) in the subject in the ultrasound image based on the high-resolution signal 24 is higher than that in the ultrasound image based on the power Doppler signals 22.

As described above, in the first embodiment, the power Doppler signals 22 representing the plural frames are input as the data based on the first ultrasound data, into the trained model 200, and the high-resolution signal representing a single frame and higher in spatial resolution of the fluid in the subject than the first ultrasound data is obtained from the trained model 200. Processing for extracting an extracted signal 23 from a power Doppler signal 22 illustrated in FIG. 3 for each frame is thereby not required and the amount of computation for obtaining the high-resolution signal 24 can be minimized, and the real-time characteristic of the high-resolution signal 24 can be maintained. That is, the ultrasonic diagnosis device 10 according to the embodiment enables obtainment of a high-resolution signal with a smaller computation load as compared to a case where normal processing is performed with respect to each frame.

### Second Embodiment

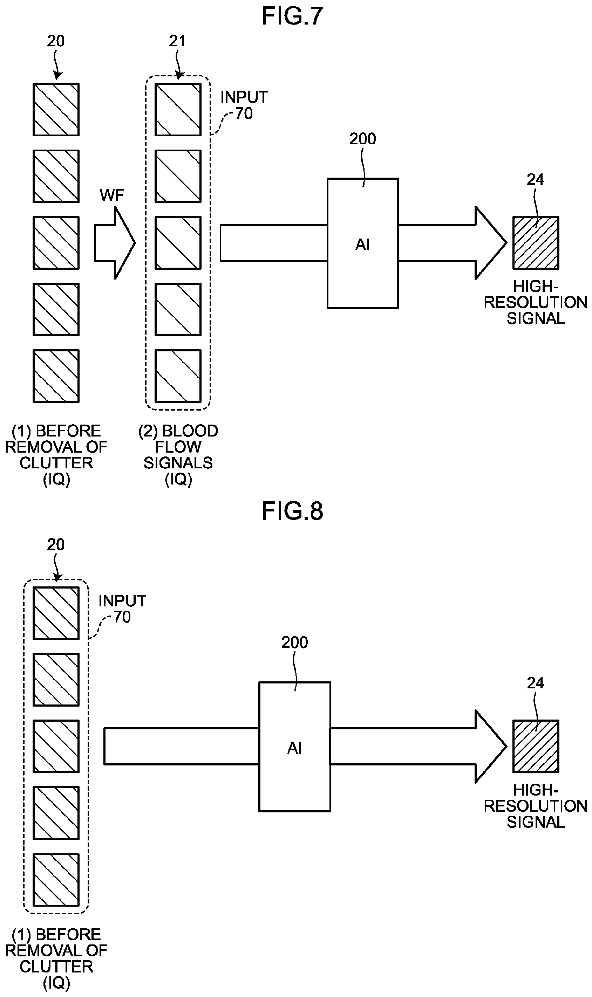

The case where power Doppler signals 22 (power Doppler data) corresponding to plural frames are used as input data for a trained model 200 has been described with respect to the first embodiment, but the input data for the trained model 200 are not to be limited to this case. For example, the second obtainment function 113 may input, as first ultrasound data, at least one of: (1) IQ data (data corresponding to the IQ signals 20 illustrated in FIG. 3) before application of a wall filter, the IQ data corresponding to plural frames; (2) Doppler data (data corresponding to the blood flow signals 21 illustrated in FIG. 3) that are the IQ data after the application of the wall filter; (3) power Doppler data (data corresponding to the power Doppler signals 22 illustrated in FIG. 3) representing absolute values of the Doppler data; and (4) data (data corresponding to the extracted signals 23 illustrated in FIG. 3) resulting from local peak extraction processing of the power Doppler data, to a trained model 200. In a case described with respect to a second embodiment, a trained model 200 is a trained model that has been trained with blood flow signals 21 serving as input data. Processing to generate a high-resolution signal 24 performed by an ultrasonic diagnosis device 10 according to the second embodiment will be described by use of FIG. 7. Redundant description of any processing similar to that of the embodiment described already will be omitted.

In the second embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. The first ultrasound data obtained on the basis of the result of the ultrasound scan of the subject are, for example, IQ signals 20 before removal of clutter, and the second ultrasound data are, for example, a high-resolution signal 24. Furthermore, in this second embodiment, as illustrated in FIG. 7, the data input to the trained model 200 and obtained on the basis of the first ultrasound data are blood flow signals 21. That is, in the second embodiment, a trained model 200 is this trained model that has been trained with the blood flow signals 21 serving as input data.

Processing in a training phase will hereinafter be described for a case where the trained model 200 is a neural network. In this case, the trained model 200 performs training of the neural network using, as data sets of supervised data used in the training, plural data sets that are each a set of blood flow signals 21 and a high-resolution signal 24 corresponding to the blood flow signals 21. Performing the training of the neural network herein means determining weight coefficients of the trained model 200 and processing circuitry 110 determines, by means of a training function not illustrated in the drawings, the weight coefficients by calculating gradients of weights using backpropagation to minimize a given objective function, for example.

In response to the above described training of the trained model 200, the processing circuitry 110 stores the determined weight coefficients of the neural network into a memory 132.

Next, to explain the processing upon execution of the trained model 200, the processing circuitry 110 calls, by means of the first obtainment function 111, the determined weight coefficients of the neural network from the memory 132. By inputting blood flow signals 21 into the trained model 200, the second obtainment function 113 obtains, as an output result from the trained model 200 and as second ultrasound data, a high-resolution signal 24 that is ultrasound data corresponding to data resulting from combination processing of data resulting from local peak extraction processing and corresponding to plural frames, from the trained model.

As described above, in the second embodiment, processing to obtain a high-resolution signal 24 from blood flow signals 21 through power Doppler signals 22 and extracted signals 23 is implemented using the trained model 200 having the blood flow signals 21 as input and the high-resolution signal 24 as output. In this case, the second obtainment function 113 inputs, as the data based on the first ultrasound data, Doppler data that are IQ data that a wall filter has been applied to, into the trained model 200. Processing to generate power Doppler signals 22 and extracted signals 23 can thereby be omitted, the amount of computation can thus be reduced, and the real-time performance of the ultrasonic diagnosis device 10 is able to be maintained. That is, a high-resolution signal is able to be obtained with a smaller computation load as compared to a case where normal processing is performed with respect to each frame. In addition, in this second embodiment, the trained model 200 generates the high-resolution signal 24 with input that is the blood flow signals 21 including phase information. The processing circuitry 110 is thereby able to obtain the high-resolution signal 24 by utilizing the phase information.

### Third Embodiment

The case where blood flow signals 21 are used as input data for a trained model 200 has been described with respect to the second embodiment. However, embodiments are not to be limited to this case. In a case described with respect to a third embodiment, a trained model 200 is a trained model that has been trained with input data that are IQ signals 20 before removal of clutter. Processing to generate a high-resolution signal 24 performed by an ultrasonic diagnosis device 10 according to the third embodiment will be described by use of FIG. 8. Redundant description of any processing similar to that of the embodiments described already will be omitted.

In the third embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. In the third embodiment, the first ultrasound data obtained on the basis of the result of the ultrasound scan of the subject are, for example, IQ signals 20 (data corresponding to the IQ signals 20 illustrated in FIG. 3) before removal of clutter and the second ultrasound data are, for example, a high-resolution signal 24. Furthermore, as illustrated in FIG. 8, in the third embodiment, the data input to the trained model 200 and obtained on the basis of the first ultrasound data are, for example, blood flow IQ signals that are the IQ signals 20 themselves before the removal of clutter. That is, in the third embodiment, the trained model 200 is a trained model that has been trained with input data that are the IQ signals 20 before the removal of clutter.

Processing in a training phase will hereinafter be described for a case where the trained model 200 is a neural network. In this case, the trained model 200 performs training of the neural network using, as data sets of supervised data used in the training, plural data sets that are each a set of IQ signals 20 before removal of clutter and a high-resolution signal 24 corresponding to the IQ signals 20 before the removal of clutter. By means of a training function not illustrated in the drawings, processing circuitry 110 determines weight coefficients by calculating gradients of weights using backpropagation to minimize a given objective function, for example.

In response to the above described training of the trained model 200, the processing circuitry 110 stores the determined weight coefficients of the neural network into a memory 132.

As to description of processing upon execution of the trained model 200 next, the processing circuitry 110 calls, by means of the first obtainment function 111, the determined weight coefficients of the neural network from the memory 132. By inputting the IQ signals 20 before the removal of clutter into the trained model 200, the second obtainment function 113 obtains, as an output result from the trained model 200 and as the second ultrasound data, a high-resolution signal 24 that is ultrasound data corresponding to data resulting from combination processing of data corresponding to plural frames and resulting from local peak extraction processing, from the trained model.

As described above, in the third embodiment, processing to obtain, from IQ signals 20 before removal of clutter, a high-resolution signal 24 through blood flow signals 21, power Doppler signals 22, and extracted signals 23 is implemented using the trained model 200 having, as input, the IQ signals 20 before the removal of clutter, and as output, the high-resolution signal 24. In this case, the second obtainment function 113 inputs, as the first ultrasound data, IQ data before application of a wall filter, into the trained model. Processing to generate blood flow signals 21, power Doppler signals 22, and extracted signals 23 is thereby able to be omitted, the amount of computation is thus able to be reduced, and the real-time performance of the ultrasonic diagnosis device 10 is thus able to be maintained. That is, a high-resolution signal is able to be obtained with a smaller computation load as compared to a case where ordinary processing is performed with respect to each frame. In addition, in this third embodiment, the trained model 200 generates the high-resolution signal 24 with input that is the IQ signals 20 before the removal of clutter. The processing circuitry 110 thereby enables incorporation of clutter removal processing into processing by the trained model 200.

### Fourth Embodiment

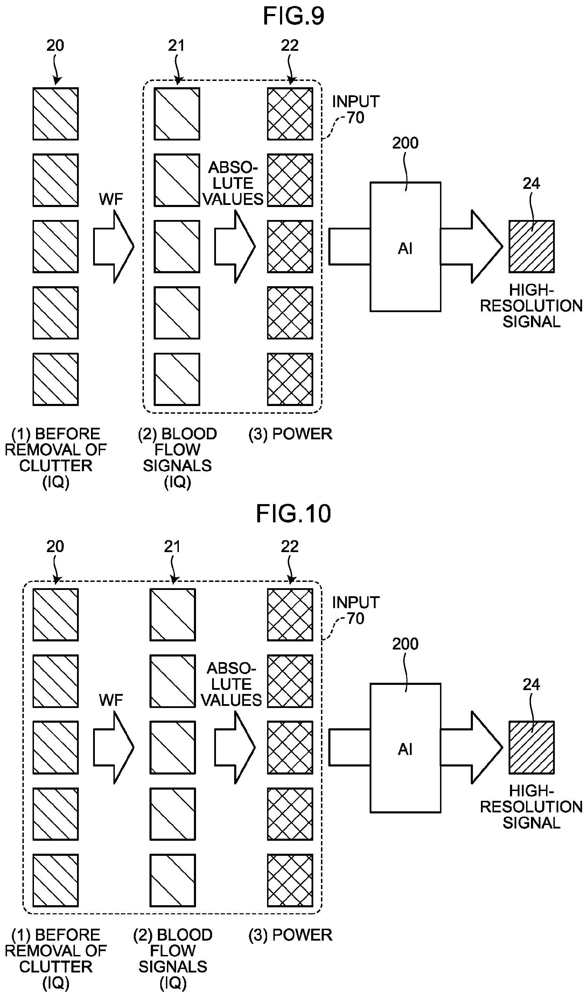

The case where one type of data is used as input data for a trained model 200 has been described with respect to the first to third embodiments. However, the embodiments are not to be limited to this case.
A case where plural types of data serve as input 70 for a trained model 200 will be described with respect to a fourth embodiment. That is, a second obtainment function 113 may input, as first ultrasound data, data that are a combination of two or more of: (1) IQ data (data corresponding to the IQ signals 20 illustrated in FIG. 3) before application of a wall filter and corresponding to plural frames; (2) Doppler data (data corresponding to the blood flow signals 21 illustrated in FIG. 3) that are the IQ data after the application of the wall filter; (3) power Doppler data (data corresponding to the power Doppler signals 22 illustrated in FIG. 3) representing absolute values of the Doppler data; and (4) data (data corresponding to the extracted signals 23 illustrated in FIG. 3) resulting from local peak extraction processing of the power Doppler data, into the trained model 200. A case where the trained model 200 is a trained model that has been trained with input data that are power Doppler signals 22 and blood flow signals 21 will be described with respect to the fourth embodiment. Processing to generate a high-resolution signal 24 performed by an ultrasonic diagnosis device 10 according to the fourth embodiment will be described by use of FIG. 9. Redundant description of any processing similar to that of the embodiments described already will be omitted.

In the fourth embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. In this fourth embodiment, the first ultrasound data obtained on the basis of the result of the ultrasound scan of the subject are, for example, IQ signals 20 before removal of clutter, and the second ultrasound data are, for example, a high-resolution signal 24. Furthermore, in this fourth embodiment, as illustrated in FIG. 9, the data input to the trained model 200 and obtained on the basis of the first ultrasound data are, for example, blood flow signals 21 and power Doppler signals 22. That is, in the fourth embodiment, the trained model 200 is a trained model that has been trained with the blood flow signals 21 and power Doppler signals 22 serving as input data.

Processing in a training phase will hereinafter be described for a case where the trained model 200 is a neural network. In this case, the trained model 200 performs training of the neural network using, as data sets of supervised data used in the training, plural data sets that are each a set of blood flow signals 21, power Doppler signals 22, and a high-resolution signal 24 corresponding to the blood flow signals 21 and power Doppler signals 22. By means of a training function not illustrated in the drawings, processing circuitry 110 determines weight coefficients by calculating gradients of weights using backpropagation to minimize a given objective function, for example.

In an example of a configuration of the neural network of the trained model 200, for example, a first layer may be connected to an input layer related to the blood flow signals 21, a second layer may be connected to an input layer related to the power Doppler signals 22, a third layer may be connected to the first layer and second layer, and an (n+1)-th layer may be connected to an n-th layer, where n is a natural number equal to or larger than 3.

Furthermore, in another example of the configuration of the neural network of the trained model 200, for example, a first layer may be connected to one of an input layer related to the blood flow signals 21 and an input layer related to the power Doppler signals 22, and an (n+1)-th layer may be connected to an n-th layer, where n is a natural number equal to or larger than 2, and at the same time, the other one of the input layer related to the blood flow signals 21 and the input layer related to the power Doppler signals 22 may be connected to all layers.

Furthermore, in yet another example of the configuration of the neural network of the trained model 200, both an input layer related to the blood flow signals 21 and an input layer related to the power Doppler signals 22 may be connected to a first layer, which is a hidden layer.

In response to the training of the trained model 200, the processing circuitry 110 stores the determined weight coefficients of the neural network into a memory 132.

As to description of processing upon execution of the trained model 200 next, the processing circuitry 110 calls, by means of the first obtainment function 111, the determined weight coefficients of the neural network from the memory 132. By inputting, as input 70, blood flow signals 21 and power Doppler signals 22 into the trained model 200, the second obtainment function 113 obtains, as an output result from the trained model 200 and as the second ultrasound data, a high-resolution signal 24 that is ultrasound data corresponding to data resulting from combination processing of data corresponding to plural frames and resulting from local peak extraction processing, from the trained model.

As described above, in the fourth embodiment, a high-resolution signal is generated using the trained model 200 having, as input, blood flow signals 21 and power Doppler signals 22 and, as output, a high-resolution signal 24. In this case, the second obtainment function 113 inputs, as the first ultrasound data, data corresponding to plural frames and being a combination of Doppler data and power Doppler data, into the trained model. The fourth embodiment has the advantages of the first embodiment and the advantages of the second embodiment, enables reduction in the amount of computation and utilization of phase information, and thus signals that are output result in high images.

### Fifth Embodiment

The case where a trained model 200 is a trained model that has been trained with input 70 that is power Doppler signals 22 and blood flow signals 21 has been described with respect to the fourth embodiment. In a fifth embodiment, a trained model 200 may be a trained model that has been trained with input data that are IQ signals 20 before removal of clutter, power Doppler signals 22, and blood flow signals 21. Processing to generate a high-resolution signal 24 performed by an ultrasonic diagnosis device 10 according to the fifth embodiment will be described by use of FIG. 10.

In the fifth embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. In this fifth embodiment, the first ultrasound data obtained on the basis of the result of the ultrasound scan of the subject are, for example, IQ signals 20 before removal of clutter, and the second ultrasound data are, for example, a high-resolution signal 24. Furthermore, as illustrated in FIG. 10, in the fifth embodiment, the data input to the trained model 200 and obtained on the basis of the first ultrasound data are, for example, the IQ signals 20 before the removal of clutter, blood flow signals 21, and power Doppler signals 22. That is, in the fifth embodiment, the trained model 200 is a trained model that has been trained with input 70 that is the IQ signals 20 before the removal of clutter, the blood flow signals 21 and the power Doppler signals 22.

Processing in a training phase will hereinafter be described for a case where the trained model 200 is a neural network. In this case, the trained model 200 performs training of the neural network using, as data sets of supervised data used in the training, plural data sets that are each a set of IQ signals 20 before removal of clutter, blood flow signals 21, power Doppler signals 22, and a high-resolution signal 24 corresponding thereto. By means of a training function not illustrated in the drawings, processing circuitry 110 determines weight coefficients by calculating gradients of weights using backpropagation to minimize a given objective function, for example.

In an example of a configuration of the neural network of the trained model 200, for example, a first layer may be connected to an input layer related to the blood flow signals 21, a second layer may be connected to an input layer related to the power Doppler signals 22, a third layer may be connected to an input layer related to the IQ signals 20 before the removal of clutter, the fourth layer may be connected to the first layer, second layer, and third layer, and an (n+1)-th layer may be connected to an n-th layer, where n is a natural number equal to or larger than 4.

Furthermore, in another example of the configuration of the neural network of the trained model 200, for example, a first layer may be connected to one or more of an input layer related to the IQ signals 20 before the removal of clutter, an input layer related to the blood flow signals 21, and an input layer related to the power Doppler signals 22, and an (n+1)-th layer may be connected to an n-th layer, where n is a natural number equal to or larger than 2, and at the same time, the other one or ones of the input layer related to the IQ signals 20 before the removal of clutter, the input layer related to the blood flow signals 21, and the input layer related to the power Doppler signals 22 may be connected to all layers.

In yet another example of the configuration of the neural network of the trained model 200, all of an input layer before the removal of clutter, an input layer related to the blood flow signals 21, and an input layer related to the power Doppler signals 22 may be connected to a first layer, which is a hidden layer.

In response to the training of the trained model 200, the processing circuitry 110 stores the determined weight coefficients of the neural network into a memory 132.

As to description of processing upon execution of the trained model 200 next, the processing circuitry 110 calls, by means of the first obtainment function 111, the determined weight coefficients of the neural network from the memory 132. By inputting, as input 70, IQ signals 20 before removal of clutter, blood flow signals 21, and power Doppler signals 22 into the trained model 200, the second obtainment function 113 obtains, as an output result from the trained model 200 and as the second ultrasound data, a high-resolution signal 24 that is ultrasound data corresponding to data resulting from combination processing of data corresponding to plural frames and resulting from local peak extraction processing, from the trained model.

As described above, in the fifth embodiment, a high-resolution signal is generated using the trained model 200 having, as input 70, IQ signals 20 before removal of clutter, blood flow signals 21, and power Doppler signals 22, and as output, a high-resolution signal 24. In this case, the second obtainment function 113 inputs, as the first ultrasound data, data corresponding to plural frames and being a combination of IQ data, Doppler data, and power Doppler data, into the trained model. The fifth embodiment has the advantages of the first embodiment, the advantages of the second embodiment, and the advantages of the third embodiment, enables reduction in the amount of computation, utilization of phase information, and inclusion of processing, such as motion correction using tissue signals, in processing by the trained model 200.

### Sixth Embodiment

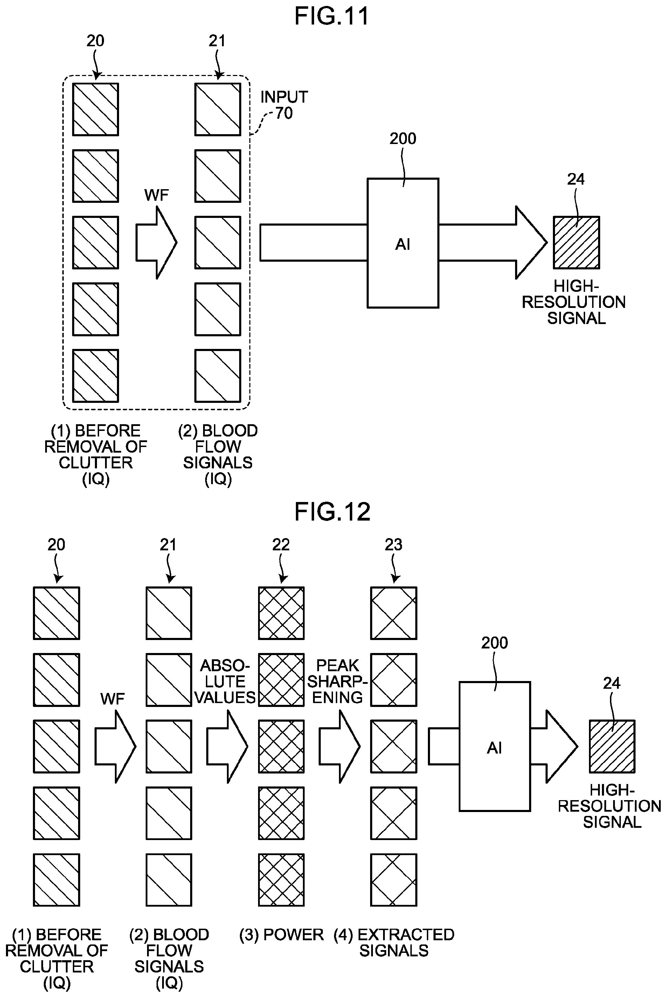

The case where a trained model 200 is a trained model that has been trained with input 70 that is power Doppler signals 22 and blood flow signals 21 has been described with respect to the fourth embodiment. In a case described with respect to a sixth embodiment, a trained model 200 is a trained model that has been trained with input data that are IQ signals 20 before removal of clutter and blood flow signals 21. Processing to generate a high-resolution signal 24 performed by an ultrasonic diagnosis device 10 according to the sixth embodiment will be described by use of FIG. 11.

In the sixth embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. In this sixth embodiment, the first ultrasound data obtained on the basis of the result of the ultrasound scan of the subject are, for example, IQ signals 20 before removal of clutter, and the second ultrasound data are, for example, a high-resolution signal 24. Furthermore, as illustrated in FIG. 11, in the sixth embodiment, the data input to the trained model 200 and obtained on the basis of the first ultrasound data are, for example, the IQ signals before the removal of clutter and blood flow signals 21. That is, in the sixth embodiment, the trained model 200 is a trained model that has been trained with input 70 that is the IQ signals 20 before the removal of clutter and the blood flow signals 21.

Processing in a training phase will hereinafter be described for a case where the trained model 200 is a neural network. In this case, the trained model 200 performs training of the neural network using, as data sets of supervised data used in the training, plural data sets that are each a set of IQ signals 20 before removal of clutter, blood flow signals 21, and a high-resolution signal 24 corresponding thereto. By means of a training function not illustrated in the drawings, processing circuitry 110 determines weight coefficients by calculating gradients of weights using backpropagation to minimize a given objective function, for example.

In an example of a configuration of the neural network of the trained model 200, for example, a first layer may be connected to an input layer related to the IQ signals 20 before the removal of clutter, a second layer may be connected to an input layer related to the blood flow signals 21, a third layer may be connected to the first layer and second layer, and an (n+1)-th layer may be connected to an n-th layer, where n is a natural number equal to or larger than 3.

Furthermore, in another example of the configuration of the neural network of the trained model 200, for example, a first layer may be connected to one of an input layer related to the IQ signals 20 before the removal of clutter and an input layer related to the blood flow signals 21, and an (n+1)-th layer may be connected to an n-th layer, where n is a natural number equal to or larger than 2, and at the same time, the other one of the input layer related to the IQ signals 20 before the removal of clutter and the input layer related to the blood flow signals 21 may be connected to all layers.

In yet another example of the configuration of the neural network of the trained model 200, both an input layer related to the IQ signals 20 before the removal of clutter and an input layer related to the blood flow signals 21 may be connected to a first layer, which is a hidden layer.

In response to the training of the trained model 200, the processing circuitry 110 stores the determined weight coefficients of the neural network into a memory 132.

As to description of processing upon execution of the trained model 200 next, the processing circuitry 110 calls, by means of the first obtainment function 111, the determined weight coefficients of the neural network from the memory 132. By inputting IQ signals 20 before removal of clutter and blood flow signals 21 into the trained model 200, the second obtainment function 113 obtains, as an output result from the trained model 200 and as the second ultrasound data, a high-resolution signal 24 that is ultrasound data corresponding to data resulting from combination processing of data corresponding to plural frames and resulting from local peak extraction processing, from the trained model.

As described above, in the sixth embodiment, a high-resolution signal is generated using the trained model 200 having, as input, IQ signals 20 before removal of clutter and blood flow signals 21, and as output, a high-resolution signal 24. In this case, the second obtainment function 113 inputs, as the first ultrasound data, data corresponding to plural frames and being a combination of IQ data and Doppler data, into the trained model. The sixth embodiment has the advantages of the second embodiment and the advantages of the third embodiment, enables utilization of phase information, and enables inclusion of, for example, motion correction using tissue signals in processing by the trained model 200.

### Seventh Embodiment

A case where a trained model 200 is a trained model that has been trained with input data that are extracted signals obtained by peak sharpening processing of power Doppler signals will be described with respect to a seventh embodiment. Processing to generate a high-resolution signal 24 performed by an ultrasonic diagnosis device 10 according to the seventh embodiment will be described by use of FIG. 12.

In the seventh embodiment, a first obtainment function 111 obtains first ultrasound data obtained on the basis of a result of an ultrasound scan of a subject, and a second obtainment function 113 obtains second ultrasound data higher in definition than the first ultrasound data from a trained model by inputting data obtained on the basis of the first ultrasound data into the trained model. The first ultrasound data obtained on the basis of the result of the ultrasound scan of the subject are, for example, IQ signals 20 before removal of clutter, and the second ultrasound data are, for example, a high-resolution signal 24. Furthermore, as illustrated in FIG. 12, in the seventh embodiment, the data input to the trained model 200 and obtained on the basis of first ultrasound data are extracted signals 23 obtained by, for example, peak sharpening processing through processing, such as multiplication of power Doppler signals by a power function. That is, in the seventh embodiment, the trained model 200 is a trained model that has been trained with input data that are the extracted signals 23.

That is, in the seventh embodiment, the trained model 200 has extracted signals 23 for respective frames as input and a high-resolution signal 24 as output. In this case, the second obtainment function 113 inputs, as the first ultrasound data, data resulting from local peak extraction processing of power Doppler data, into the trained model. That is, the extracted signals 23 are input respectively for the plural frames into the trained model 200. The high-resolution signal 24 is considered to be the linear sum of the extracted signals 23, and optimizing the weight coefficients with a model using AI is considered to enable adaptive adjustment of weights for the respective frames and obtainment of the high-resolution signal 24.

Processing in a training phase will hereinafter be described for a case where the trained model 200 is a neural network. In this case, the trained model 200 performs training of the neural network using, as data sets of supervised data used in the training, plural data sets that are each a set of extracted signals 23 and a high-resolution signal 24 corresponding to the extracted signals 23. Performing the training of the neural network herein means determining model weight coefficients of the trained model 200 and processing circuitry 110 determines, by means of a training function not illustrated in the drawings, the weight coefficients by calculating gradients of weights using backpropagation to minimize a given objective function, for example.

In response to the above described training of the trained model 200, the processing circuitry 110 stores the determined weight coefficients of the neural network into a memory 132.

As to description of processing upon execution of the trained model 200 next, the processing circuitry 110 calls, by means of the first obtainment function 111, the determined weight coefficients of the neural network from the memory 132. By inputting extracted signals 23 to the trained model 200, the second obtainment function 113 obtains, as an output result from the trained model 200 and as the second ultrasound data, a high-resolution signal 24 that is ultrasound data corresponding to data resulting from combination processing of data corresponding to plural frames and resulting from local peak extraction processing, from the trained model.

As described above, in the seventh embodiment, the trained model 200 is trained with input data that are extracted signals 23 obtained by peak sharpening processing of power Doppler signals. Weights for the respective frames are thereby able to be adaptively adjusted. The peak sharpening processing is not to be limited to the case where the multiplication by a power function is used and may be processing to extract or enhance local peaks. In any case, the high-resolution signal 24 is higher in spatial resolution of fluid than the extracted signals 23.

### Eighth Embodiment

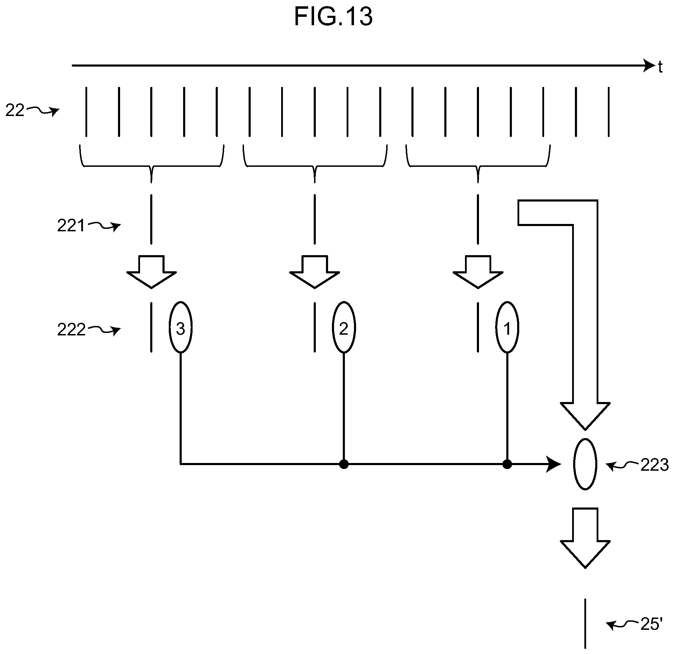

In the example described with respect to the first embodiment, signals (extracted signals 23) obtained by peak sharpening of power Doppler signals 22 are input data (data based on first ultrasound data) for a trained model 200 and a high-resolution signal 24 corresponding to a signal representing a single frame is output data (second ultrasound data) from the trained model 200, the signal being obtained by combining the extracted signals 23 corresponding to plural frames. However, the data based on the first ultrasound data, and the second ultrasound data may be other data. For example, as illustrated in FIG. 13, power Doppler signals 22 representing plural frames may be input data (data based on first ultrasound data) for a trained model 200, and a high-definition signal 25 corresponding to a high-definition signal 25' representing a single frame may be output data (second ultrasound data) from the trained model 200, the high-definition signal 25' being obtained by processing of the power Doppler signals 22, the processing being illustrated in FIG. 13.

The following description is on the processing illustrated in FIG. 13. The power Doppler signals 22 are similar to those of the first embodiment and are signals obtained by finding absolute values of IQ signals (Doppler data) serving as blood flow signals, the signals representing plural frames. The power Doppler signals 22 are added up into added signals 221 each corresponding to plural frames (five frames in FIG. 13). The added signals 221 each represent a single frame improved in SN ratio as compared to the power Doppler signals 22. The added signals 221 corresponding to plural frames are generated from the power Doppler signals 22 consecutive in a time direction (FIG. 13 illustrates the added signals 221 representing three frames).

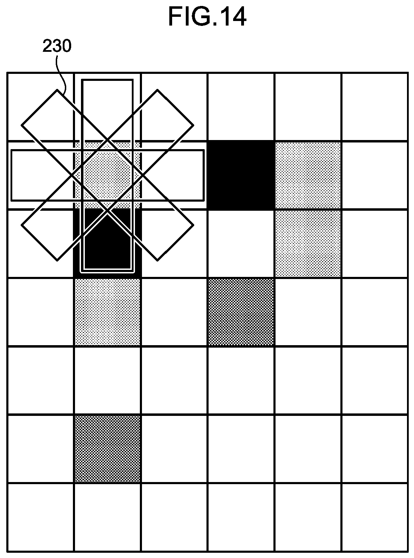

Thereafter, from the added signals 221 representing the plural frames, sets of identification information 222 corresponding to these frame are generated. A set of identification information 222 is information indicating, for each position in a frame represented by an added signal 221, a magnitude relation between a signal value corresponding to that position and signal values corresponding to surroundings of the position. For example, as illustrated in FIG. 14, a kernel 230 is placed at a position of interest in a frame (image) represented by an added signal 221, and a signal value corresponding the position of interest and a signal value corresponding to each position in a range (surrounding the position of interest) where the kernel 230 has been placed are compared with each other for a magnitude relation therebetween. Information indicating whether or not each position in the frame has a signal value larger than signal values corresponding to surroundings of that position is then obtained as a set of identification information 222.

Specifically, in a case where the kernel 230 used is based on a 3 (vertical) by 1 (horizontal) ratio, the center (second element) of the kernel 230 is placed at a position of interest and whether a signal value corresponding to the position is larger than signal values corresponding to positions at both ends (first element and third element) of the kernel 230 is determined. In a case where the signal value corresponding to the position of interest is larger than the signal values corresponding to the positions around the position of interest, the position of interest is represented by "1", for example. On the contrary, in a case where the signal value corresponding to the position of interest is smaller than the signal values corresponding to the positions around the position of interest, the position of interest is represented by "0", for example. Such processing is performed at each position of the frame and the set of identification information 222 for the added signal 221 is thereby extracted. The set of identification information 222 is a binary image having each position (each pixel) represented by "0" or "1", the position being of the frame represented by the added signal 221 and is information (local peak position information) for identifying, per frame, positions where fluid (a blood flow) is present in a local range.

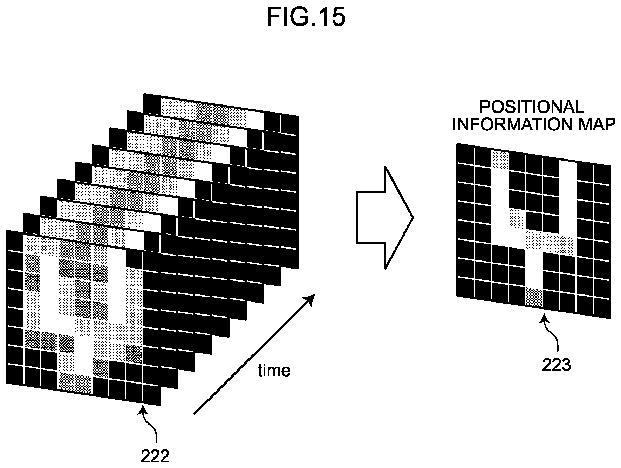

As illustrated in FIG. 15, a positional information map 223 is generated by analysis of the plural sets of identification information 222. The positional information map 223 is information statistically representing positions in a frame, the positions being where the fluid is present in a time period corresponding to the plural frames (FIG. 13 illustrating three frames and FIG. 15 illustrating nine frames). For example, the larger the number of positions that are "1", the positions corresponding to one another among the plural sets of identification information 222, the larger the numerical value at that position, and the larger the number of positions that are "0", the smaller the numerical value at that position. Weight coefficients at the respective positions of the positional information map 223 are thereby set.

On the basis of an added signal 221 representing the latest frame of the plural frames and the positional information map 223, the high-definition signal 25' representing a single frame is then generated. The high-definition signal 25' is a signal that is higher in spatial resolution of the fluid in the subject than the power Doppler signals 22 and higher in definition of the fluid flowing in the subject (depicts the fluid with high definition).

As described above, the method illustrated in FIG. 13 requires plural sets of processing for the high-resolution signal 25' to be obtained. Therefore, when a large number of frames are used, the real-time characteristic of high-definition ultrasound data may be degraded. In particular, obtaining the sets of identification information 222 and the positional information map 223 may take time according to the number of frames and the real-time characteristic of the high-resolution signal 25' may be degraded.

An ultrasonic diagnosis device 10 according to this embodiment outputs high-definition ultrasound data by using a trained model based on artificial intelligence (AI) and thereby reduces the amount of computation and maintains the real-time performance required for ultrasound diagnosis.

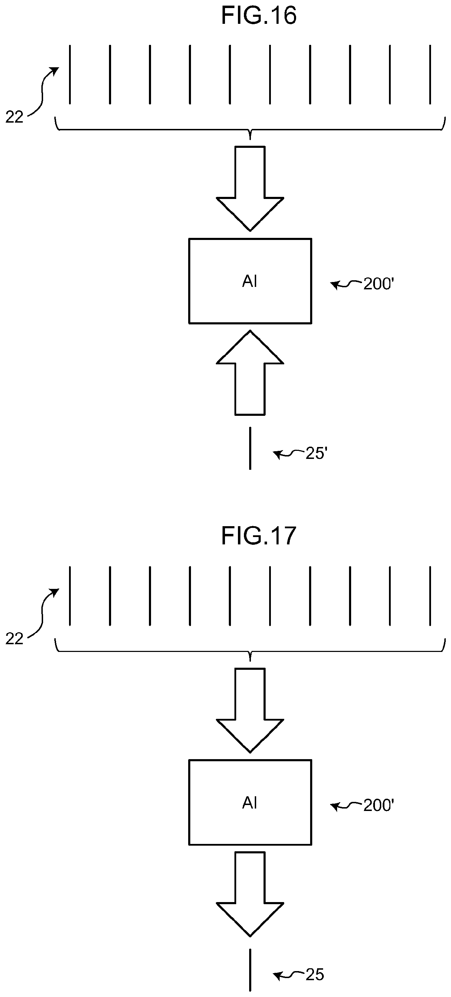

Processing in a training phase will be described hereinafter. For example, as illustrated in FIG. 16, training of a neural network is performed with data sets each having power Doppler signals 22 representing plural frames and serving as input data, and high-definition data (a high-resolution signal 25') representing a single frame and serving as output data. In the training of the neural network, model weight coefficients of a trained model 200 are determined. The weight coefficients are determined, by means of a training function not illustrated in the drawings, by calculation of gradients of weights using backpropagation to minimize a given objective function, for example.

Processing in an operational phase (inference phase) will be described next by reference to FIG. 17. A first obtainment function 111 (first obtainment unit) obtains first ultrasound data obtained on the basis of a result of an ultrasound scan of a subject. The first ultrasound data are obtained by execution of the ultrasound scan of the subject via an ultrasound probe 5 and represent plural frames that are consecutive in a time direction. The first ultrasound data correspond to received signals (reflected ultrasound signals) representing the plural frames.

An extraction function 112 obtains IQ signals representing the plural frames by performing phasing addition and quadrature detection processing of the received signals (the reflected ultrasound signals) representing the plural frames. Furthermore, the extraction function 112 obtains blood flow signals 21 representing the plural frames by applying a wall filter (WF) to the IQ signals. Furthermore, the extraction function 112 obtains power Doppler signals 22 representing the plural frames by finding absolute values of the IQ signals (Doppler Data) serving as the blood flow signals.

A second obtainment function 113 inputs the power Doppler signals 22 (data based on the first ultrasound data) into a trained model 200'. Furthermore, the second obtainment function 113 obtains a high-definition signal 25 (second ultrasound data) as output from the trained model 200. The high-definition signal 25 corresponds to the high-definition signal 25' obtained by the processing illustrated in FIG. 13. An ultrasound image generated on the basis of the high-definition signal 25 has image quality equivalent to that of the ultrasound image illustrated in FIG. 6B and is higher in spatial resolution of fluid (blood flow) in the subject than the ultrasound image illustrated in FIG. 6A and based on the power Doppler signals 22.

As described above, in the eighth embodiment, the power Doppler signals 22 representing the plural frames and serving as the data based on the first ultrasound data are input to the trained model 200, and the high-resolution signal 25 representing a single frame and higher in spatial resolution of the fluid in the subject than the first ultrasound data is obtained from the trained model 200. The amount of computation for obtaining the high-resolution signal 25 is thereby able to be reduced and the real-time characteristic of the high-definition signal 25 is able to be maintained. That is, the ultrasonic diagnosis device 10 according to this embodiment enables obtainment of a high-resolution signal with a smaller computation load as compared to a case where ordinary processing is performed with respect to each frame.

### Other Embodiments

The case where the second obtainment function 113 inputs first ultrasound data to a trained model 200 has been described with respect to each of the fourth to sixth embodiments, the first ultrasound data being a combination of two or more of: (1) IQ data; (2) Doppler data; (3) power Doppler data; and (4) data that have been subjected to local peak extraction processing, the data corresponding to plural frames, but the embodiments are not to be limited to the combination mentioned above. For example, the second obtainment function 113 may input, as the first ultrasound data, data that are a combination of (1) the IQ data and (3) the power Doppler data, the data corresponding to the plural frames, into the trained model 200.

The embodiments are not to be limited to the above described examples, and a trained model 200 may be a trained model that has been trained with input data that are signals in a stage earlier than that of IQ signals before removal of clutter, for example, RF signals or channel data.

Furthermore, the above described first to eighth embodiments may be implemented after application of storage processing in a frame direction or spatial direction to: IQ signals 20 before removal of clutter; blood flow signals 21; power Doppler signals 22; extracted signals 23; and a high-resolution signal 24. In this case, the trained model 200 is a trained model that has been trained with input data that are data obtained by interpolation processing in the frame direction or spatial direction.

The case where output data from a trained model 200 is a high-resolution signal 24 has been described above but the embodiments are not to be limited to this case. For example, output data from a trained model 200 may be differential data for generating a high-resolution signal 24 or mask data representing information on data points to be subjected to correction processing performed for generating a high-resolution signal 24.

The case where a trained model 200 is a neural network has been described with respect to the embodiments, but the embodiments are not to be limited to this case, and the processing circuitry 110 may generate a high-resolution signal on the basis of input data by machine learning, such as linear regression, logistic regression, a decision tree, gradient boosting, or PCA, instead of the trained model 200.

Furthermore, first ultrasound data obtained by the first obtainment function 111 are not necessarily data obtained on the basis of a scan by the ultrasonic diagnosis device 10 and may be, for example, data received from an external workstation.

The example where high-resolution blood flow data are generated has been described mainly thus far but a method according to an embodiment also enables generation of high-resolution body tissue data or contrast imaging data.

At least one of the embodiments described above enables reduction in the amount of computation in processing of ultrasound data or obtainment of high-resolution ultrasound data.

The following notes are disclosed as one aspect and optional features of the invention, in relation to the embodiments described above.
Note 1. An ultrasonic diagnosis device provided in one aspect of the present invention includes an ultrasound probe, a first obtainment unit, and a second obtainment unit. The first obtainment unit obtains first ultrasound data obtained by execution of an ultrasound scan of a subject via the ultrasound probe, the first ultrasound data representing plural frames consecutive in a time direction. The second obtainment unit inputs data based on the first ultrasound data into a trained model, and obtains second ultrasound data from the trained model, the second ultrasound data representing a single frame and being higher in spatial resolution of fluid in the subject than the first ultrasound data.
Note 2. The second obtainment unit may input, as the first ultrasound data, at least one of: (1) IQ data before application of a wall filter and corresponding to the plural frames; (2) Doppler data that correspond to the plural frames and are the IQ data after the application of the wall filter; (3) power Doppler data corresponding to the plural frames and representing absolute values of the Doppler data; and (4) data corresponding to the plural frames and resulting from local peak extraction processing of the power Doppler data, into the trained model, and obtain, as the second ultrasound data, ultrasound data corresponding to data resulting from combination processing of data corresponding to the plural frames and resulting from local peak extraction processing, from the trained model.
Note 3. The second obtainment unit may input, as the first ultrasound data, data that are a combination of two or more of: (1) the IQ data; (2) the Doppler data; (3) the power Doppler data; and (4) the data resulting from the local peak extraction processing, into the trained model, the data corresponding to the plural frames.
Note 4. The second obtainment unit may input, as the first ultrasound data, data that are a combination of (1) the IQ data and (2) the Doppler data, into the trained model, the data corresponding to the plural frames.
Note 5. The second obtainment unit may input, as the first ultrasound data, data that are a combination of (1) the IQ data and (3) the power Doppler data, into the trained model, the data corresponding to the plural frames.
Note 6. The second obtainment unit may input, as the first ultrasound data, data that are a combination of (2) the Doppler data and (3) the power Doppler data, into the trained model, the data corresponding to the plural frames.
Note 7. The second obtainment unit may input, as the first ultrasound data, data that are a combination of (1) the IQ data, (2) the Doppler data, and (3) the power Doppler data, into the trained model, the data corresponding to the plural frames.
Note 8. A medical information processing device provided in one aspect of the present invention includes an ultrasound probe, a first obtainment unit, and a second obtainment unit. The first obtainment unit obtains first ultrasound data obtained by execution of an ultrasound scan of a subject via the ultrasound probe, the first ultrasound data representing plural frames consecutive in a time direction. The second obtainment unit inputs data based on the first ultrasound data into a trained model, and obtains second ultrasound data from the trained model, the second ultrasound data representing a single frame and being higher in spatial resolution of fluid in the subject than the first ultrasound data.
Note 9. A program provided in one aspect of the present invention causes a computer to execute a process including: obtaining first ultrasound data obtained by execution of an ultrasound scan of a subject via an ultrasound probe, the first ultrasound data representing plural frames consecutive in a time direction; inputting data based on the first ultrasound data into a trained model; and obtaining second ultrasound data from the trained model, the second ultrasound data representing a single frame and being higher in spatial resolution of fluid in the subject than the first ultrasound data.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An ultrasonic diagnosis device, comprising:
an ultrasound probe (5); and
processing circuitry(110) configured to
obtain first ultrasound data obtained by execution of an ultrasound scan of a subject via the ultrasound probe (5), the first ultrasound data representing plural frames consecutive in a time direction, and
input data based on the first ultrasound data into a trained model to obtain second ultrasound data from the trained model, the second ultrasound data representing a single frame and being higher in spatial resolution of fluid in the subject than the first ultrasound data.

2. The ultrasonic diagnosis device according to claim 1, wherein the processing circuitry(110) is configured to
input, as the first ultrasound data, at least one of: [1] IQ data corresponding to the plural frames and before application of a wall filter; [2] Doppler data that correspond to the plural frames and are the IQ data after the application of the wall filter; [3] power Doppler data corresponding to the plural frames and representing absolute values of the Doppler data; and [4] data corresponding to the plural frames and resulting from local peak extraction processing of the power Doppler data, into the trained model, and
obtain, as the second ultrasound data, ultrasound data corresponding to data resulting from combination processing of data corresponding to the plural frames and resulting from local peak extraction processing, from the trained model.

3. The ultrasonic diagnosis device according to claim 2, wherein the processing circuitry(110) is configured to input, as the first ultrasound data, data that are a combination of two or more of: [1] the IQ data; [2] the Doppler data; [3] the power Doppler data; and [4] the data resulting from the local peak extraction processing, into the trained model, the data corresponding to the plural frames.

4. The ultrasonic diagnosis device according to claim 2, wherein the processing circuitry(110) is configured to input, as the first ultrasound data, data that are a combination of [1] the IQ data and [2] the Doppler data, into the trained model, the data corresponding to the plural frames.

5. The ultrasonic diagnosis device according to claim 2, wherein the processing circuitry(110) is configured to input, as the first ultrasound data, data that are a combination of [1] the IQ data and [3] the power Doppler data, into the trained model, the data corresponding to the plural frames.

6. The ultrasonic diagnosis device according to claim 2, wherein the processing circuitry(110) is configured to input, as the first ultrasound data, data that are a combination of [2] the Doppler data and [3] the power Doppler data, into the trained model, the data corresponding to the plural frames.

7. The ultrasonic diagnosis device according to claim 2, wherein the processing circuitry(110) is configured to input, as the first ultrasound data, data that are a combination of [1] the IQ data, [2] the Doppler data, and [3] the power Doppler data, into the trained model, the data corresponding to the plural frames.

8. A medical information processing device, comprising:
processing circuitry(110) configured to
obtain first ultrasound data obtained by execution of an ultrasound scan of a subject via an ultrasound probe (5), the first ultrasound data representing plural frames consecutive in a time direction, and
input data based on the first ultrasound data into a trained model to obtain second ultrasound data from the trained model, the second ultrasound data representing a single frame and being higher in spatial resolution of fluid in the subject than the first ultrasound data.

9. A program for causing a computer to execute a process including:
obtaining first ultrasound data obtained by execution of an ultrasound scan of a subject via an ultrasound probe (5), the first ultrasound data representing plural frames consecutive in a time direction; and
inputting data based on the first ultrasound data into a trained model and obtaining second ultrasound data from the trained model, the second ultrasound data representing a single frame and being higher in spatial resolution of fluid in the subject than the first ultrasound data.
